(19) Europäisches Patentamt
European Patent Office
Office européen des brevets

(11) Numéro de publication : **0 392 888 B1**

(12) # FASCICULE DE BREVET EUROPEEN

(45) Date de publication du fascicule du brevet : **09.09.92 Bulletin 92/37**

(51) Int. Cl.[5] : **A61K 35/78,** A61K 31/725, // (A61K35/78, 31:725)

(21) Numéro de dépôt : **90400614.5**

(22) Date de dépôt : **06.03.90**

(54) **Compositions thérapeutiques pour le traitement ou la prévention du ronflement.**

(30) Priorité : **12.04.89 FR 8905029**

(43) Date de publication de la demande : **17.10.90 Bulletin 90/42**

(45) Mention de la délivrance du brevet : **09.09.92 Bulletin 92/37**

(84) Etats contractants désignés : **AT BE CH DE DK ES FR GB GR IT LI NL SE**

(56) Documents cités :
**EP-A- 0 130 550**
**WO-A-87/07504**

(73) Titulaire : **Andermann, Guy**
**2, Rond-Point Esplanade**
**F-67000 Strasbourg (FR)**

(72) Inventeur : **Andermann, Guy**
**2, Rond-Point Esplanade**
**F-67000 Strasbourg (FR)**

EP 0 392 888 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a trait au domaine des solutés pour instillations nasales. Elle concerne plus particulièrement un nouveau soluté nasal destiné à la prévention ou au traitement du ronflement, ou des manifestations du ronflement.

On sait que le ronflement constitue une manifestation désagréable pour l'entourage du ronfleur. Le ronflement est un bruit qui provient d'une vibration anormale du voile du palais. Le ronfleur souffre souvent par ailleurs de l'obstruction des fosses nasales, ce qui ajoute aux troubles enregistrés.

On distingue classiquement le ronfleur du ronchopathe, qui peut être atteint d'une affection grave, le syndrôme d'apnée du sommeil (SAS).

Les traitements actuels sont constitués soit par la chirurgie (uvulopalato-pharyngoplastie), soit par des signaux électroniques qui agissent sur le cerveau, en envoyant des messages au ronfleur, ce qui le réveille et l'induit à changer de position. On a proposé également l'acuponcture, ou des traitements médicamenteux par instillation de gouttes nasales. On a utilisé respectivement des vasoconstricteurs, qui décongestionnent la muqueuse nasale (brevet U.S. 2.989.437) ou des agents tensioactifs associés ou non à de la glycérine, comme dans le brevet U.S. 4.556.557 par exemple. Les tensioactifs peuvent être des surfactifs cationiques, anioniques ou non ioniques, comme dans les produits commerciaux Phonarex$^{R}$ ou Ronflux$^{R}$, vendus dans certains pays européens.

Il s'agit de produits hydratants de la muqueuse nasale, dont la courte durée de contact avec la muqueuse limite significativement leur efficacité, sauf s'ils sont administrés un grand nombre de fois au cours de la nuit. Par ailleurs, certains de ces produits sont susceptibles de provoquer des hémorragies nasales plus ou moins graves lorsque les utilisateurs abusent de ces produits. Les vasoconstricteurs de type naphazoline, tétryzoline, xylométazoline, ou les dérivés de la phénylpropanolamine provoquent des accoutumances et des effets rebonds qui s'expriment par une certaine forme de toxicomanie.

Les tensioactifs ont été abandonnés parce qu'ils ne présentent pas les caractéristiques souhaitées pour se maintenir suffisamment en contact avec les tissus de la muqueuse nasale.

Il a maintenant été découvert que l'on pouvait pallier les inconvénients décrits ci-dessus, et mettre à la portée des ronfleurs une nouvelle composition de très grande efficacité, bien tolérée par l'organisme, et en particulier par la muqueuse nasale, capable de traiter le ronflement ou de le prévenir.

Conformément à l'invention, la nouvelle composition pour soluté nasal renferme des polymères naturels ou semi-synthétiques de haut poids moléculaire. Il s'agit de mucopolysaccharides du type glycosaminoglycanes, qui se trouvent soit dans la nature tels quels, soit sont synthétisés à partir de xylanes d'origine naturelle (dérivés semi-synthétiques). Ces xylanes peuvent être extraits de certains bois, comme le bois de hêtre par exemple, et sont ensuite transformées par synthèse en sel de sodium de polyesters sulfuriques.

C'est ainsi qu'on obtient le pentosane polysulfate décrit dans le brevet FR 1.050.576 du 6.2.1952 ou le BSM 2.227 M du 7.12.1962.

L'invention consiste essentiellement en l'utilisation de polyanions sulfatés sous forme de polymères d'origine naturelle ou d'un de leurs sels sodiques pharmaceutiquement acceptables, pour la fabrication d'un soluté nasal destiné à la prévention ou au traitement du ronflement ou de ses manifestations sonores.

Il s'agit essentiellement d'analogues de structure des mucopolysaccharides acides sulfatés naturels, tels que les acides chondroïtines sulfuriques, les keratosulfates, les dermatanes sulfates, l'héparine, ou l'acide hyaluronique. Il s'agit toujours de polyanions de structure générale:

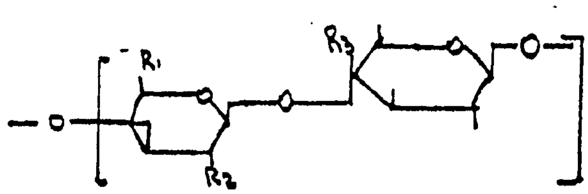

$R_1$ = H ou $-COONa$
$R_2$ = H ou $-SO_3Na$
$R_3$ = H ou $-NH-C(=O)-CH_3$

Ces substances ont des propriétés parfois comparables du point de vue de leur structure, mais souvent différentes les unes des autres du point de vue de leur activité pharmacologique. Elles sont connues sous le nom générique d'héparinoïdes, bien que la plupart d'entre elles, comme le sulfate de chondroïtine, ou le pentosane polysulfate n'aient plus d'activité anticoagulante du type de celle de l'héparine proprement dite.

On a décrit des applications thérapeutiques diverses pour chacune de ces substances. C'est ainsi que certains dérivés sont utilisés en thérapeutique comme agents antiviraux (Zbl. Bakt. Hyg.I.Abt.Orig. 1977, A 237, pp.1-34), d'autres comme des fibrinolytiques hypolipémiants comme c'est le cas du pentosane polysulfate

(Arzneim. Forsch. 1972, 22, pp. 759-763).

D'autres encore comme le sulfate de chondroïtine, ont été décrits soit comme ayant des propriétés de conservation des tissus oculaires, ou pouvant jouer le rôle d'un substituant des larmes (brevet européen 0063973). Dans ce dernier cas, on utilise préférentiellement certaines fractions de sulfate de chondroïtine (type A) plutôt que d'autres fractions (B ou C ou des mélanges A+C).

Il s'agit toujours de substances de haute viscosité, très hydrophiles, dont la structure est souvent très proche de celle de tissus naturels comme le cartilage, le tissu conjonctif, etc. Le sulfate de chondroïtine A par exemple, instillé sous forme de collyre à 3 %, a montré une importante activité mucomimétique au niveau de l'oeil de lapin, par sa capacité à conserver la surface éphithéliale au niveau des microstructures (J.Fr. Ophtalmol. 1984, 1, pp. 41-50).

La demanderesse a découvert que certains polysaccharides à poids moléculaire compris entre 1.000 et 1.000.000, exercent de façon surprenante une activité au niveau du nez, en particulier en évitant le ronflement de façon prolongée. La plupart des substances polyanioniques décrites plus haut, montrent cette efficacité. Certaines, comme l'héparine elle-même ou les héparinoides, ne peuvent être utilisés dans cette indication à cause de leurs effets systémiques gênants. C'est ainsi que l'héparine exerce une activité anticoagulante importante au niveau de la muqueuse nasale ou au niveau systémique, telle que cette substance ne pourra être utilisée dans cette indication nouvelle.

Par contre, on a trouvé que les dérivés polysulfatés de xylane, de dextrane, de polyuronides ou de mucopolysaccharides, n'ayant pas elles-mêmes aucune action anticoagulante, pouvaient particulièrement convenir au traitement ou à la prévention du ronflement, et pouvaient être administrées de façon chronique sans altérer la muqueuse nasale, et sans provoquer de toxicité systémique. Bien au contraire, il a été découvert de façon fortuite, que la voie nasale constituait une intéressante voie d'administration pour certains d'entre eux qui ont montré des propriétés hypolipémiantes.

L'invention décrit également l'utilisation d'un glycosaminoglycane sulfaté ou un de ses sels sodiques pharmaceutiquement acceptable pour la fabrication d'un soluté nasal pour la prévention ou le traitement du ronflement ou de ses manifestations sonores.

C'est ainsi que la demanderesse peut revendiquer des indications thérapeutiques nouvelles, ainsi que des modes de présentation et des voies d'administration (administration nasale) totalement différents de celles préconisés jusqu'à présent (gélules, injections, collyres).

L'invention concerne également des compositions pour prévenir ou traiter le ronflement avec des gouttes nasales administrées sous forme de soluté, qui renferment comme constituant actif le sel sodique de sulfate de chondroïtine, de préférence de poids moléculaire compris entre 1.000 et 1.000.0000.

Il s'agit essentiellement d'une poudre blanche obtenue à partir de cartilages de poissons ou de mammifères, sans odeur ni saveur, et stable dans l'eau à pH compris entre 5 et 8, à des concentrations comprises entre 1 et 10 %.

Outre les constituants actifs susmentionnés, et plus particulièrement le sulfate de chondroïtine sodique, les compositions pour soluté nasal selon l'invention, doivent renfermer divers agents de formulation, pour permettre une administration bien tolérée et confortable chez l'homme.

Ces constituants sont essentiellement des tampons, des isotonisants, des parfums, dont le rôle est connu en soi. Le choix du conservateur à incorporer dans la composition est délicat, dans la mesure où les glycosaminoglycanes de l'invention sont essentiellement des polyanions chargés négativement, comme déjà indiqué plus haut. La plupart des antiseptiques pouvant être utilisés comme conservateurs faisant partie de familles chimiques chargées positivement (comme par exemple le chlorure de benzalkonium, les ammoniums quaternaires en général, les sels de chlorhexidine etc...), le choix d'un conservateur non toxique et bien toléré par la muqueuse nasale est délicat. C'est ainsi que des dérivés comme l'Imidurée, la DMDM hydantoine auraient pu être utilisés dans la composition finale, mais leur médiocre tolérance locale les a éliminés. Les dérivés de l'acide p-hydroxybenzoique ont pu être retenus, mais ils montrent un certain pouvoir allergisant. Seuls les dérivés mercuriels comme les sels de phénylmercure (nitrate, borate, acétate) présentaient le meilleur rapport sécurité/efficacité lorsqu'ils étaient utilisés en concentration très faible (de l'ordre du mg %).

Une composition préférée selon l'invention est représentée par un soluté nasal à base de sulfate de chondroïtine sodique, caractérisé en ce que ledit soluté contient en ordre une solution aqueuse tamponnée, isotonique, contenant un conservateur compatible, de type mercuriel (nitrate ou borate phénylmercurique).

Il a en outre été trouvé de façon inattendue, que lorsque les dérivés mucopolysaccharidiques solubles dans l'eau décrits plus haut, étaient associés à diverses substances d'origine végétale, l'efficacité clinique de la composition finale pouvait significativement augmenter.
Il s'agit là d'un cas de synergie pharmaco-thérapeutique particulièrement intéressant.

Ces substances végétales, parmi lesquelles on peut citer Belladonna, Sambucus, Bryonia, sont elles-mêmes synergisées par différents constituants minéraux, utilisés à des concentrations homéopathiques. De

préférence, les substances végétales elles-mêmes peuvent être diluées à des concentrations faibles, comme celles utilisées en homéopathie (1 CH à 100 CH).

Les solutions aqueuses convenant à l'administration nasale, peuvent contenir environ 0,1 % à 10 % en poids d'un sel de sodium soluble de polysaccharide, comme par exemple le pentosane polysulfate, le sulfate de chondroïtine, ou l'acide hyaluronique.
Il est recommandé d'appliquer ces solutions une seule fois avant l'endormissement, mais les applications peuvent être répétées plusieurs fois par nuit, si nécessaire.

L'invention sera mieux comprise par la description ci-dessous, relative d'une part à une série d'exemples de solutés nasals non limitatifs, aptes à convenir au but recherché, et d'autre part, aux résultats de travaux cliniques réalisés avec de telles compositions, ou au mieux avec une composition type, à base d'un des mucopolysaccharides choisis parmi ceux qui ont été décrits dans l'invention.

1. Exemples de compositions selon l'invention

Exemple 1

| | |
|---|---|
| Sel sodique de pentosane polysulfate | 5,00 g |
| Chlorure de sodium | 0,37 g |
| Tampon phosphate (à pH 7±0,5) | 0,35 g |
| Borate phénylmercurique | 0,003g |
| Eau purifiée qsp | 100 ml |

Exemple 2

| | |
|---|---|
| Sel sodique d'acide hyaluronique | 4,00 g |
| Acide borique | 1,200g |
| Tampon phosphate (à pH 7±0,5) | 0,35 g |
| Borate phénylmercurique | 0,002g |
| Eau purifiée qsp | 100 ml |

Exemple 3

| | |
|---|---|
| Sel sodique d'acide hyaluronique | 3,00 g |
| Acide borique | 0,10 g |
| Tampon phosphate (à pH 7±0,5) | 0,25 g |
| Borate phénylmercurique | 0,37 g |
| Eau purifiée qsp | 100 ml |

Exemple 4

Identique à l'exemple 3, si ce n'est qu'on y rajoute un mélange homéopathique minéral et végétal composé de Alumina, Belladonna Bryona, Plumbum, Sambucus à 5 CH, et de l'eau de rose à 5 %.

Ainsi, comme on peut le voir, des aromes, des tampons, des produits d'origine végétale et des antiseptiques conservateurs peuvent être rajoutés au principe actif de la composition.

2. Expérimentation clinique

L'objectif de cet essai consiste à étudier l'efficacité du soluté nasal, objet de l'invention, dans la diminution ou la suppression des symptômes du ronflement.

80 patients ont été recrutés pour cet essai clinique. Ce nombre relativement élevé de patients est justifié par une désaffection connue pour le traitement de cette affectation.
En effet, du fait de la particularité de cette maladie, dont le traitement vise à supprimer un symptôme dont le patient ne se plaint pas directement, celui-ci a tendance à ne pas se considérer pas comme malade.

Il n'y a pas eu de critère d'exclusion d'âge, de sexe ou de profession pour le recrutement des volontaires. Tous les volontaires ont eu les caractéristiques suivantes :

1. Etre reconnus par eux-mêmes et par leur partenaire conjugal comme étant des ronfleurs suffisamment bruyants pour occasionner des dérangements nocturnes du fait de leur ronflement.
2. Ne pas être porteur d'une infection rhinopharyngée
3. Ne pas être porteur de déviation de la cloison nasale
4. Dormir régulièrement avec le même partenaire, ce dernier étant seul capable de participer à l'évaluation de l'efficacité du traitement.

Les malades obèses (>100 kg) et porteurs de troubles organiques (comme par exemple un pharynx étroit) ont été exclus du recrutement de l'essai.

Aucun traitement simultané n'a été autorisé pour autant que son but a été le même que celui du soluté nasal à tester. Les malades ayant subi des traitements préalables dans la même indication, ont été acceptés.

Le produit testé était celui de l'exemple 3

Mode d'emploi du produit

Le produit a été administré quelques minutes avant l'endormissement. Si le sommeil a été interrompu, ou si le ronflement a repris au cours de la nuit, une seconde administration, ou même une troisième, ont été recommandées.

Le mode d'emploi suivant accompagnait les échantillons distribués aux patients :
". En position allongée sur le dos, introduire un jet de soluté dans chaque narine.
. Pincer les deux narines pendant une minute environ pour favoriser le contact avec les muqueuses.

Les malades ont été examinés et interrogés :
– avant l'administration du produit
– après 1 mois d'administration du produit
– après 3 mois d'administration du produit

A chaque examen, les patients ont reçu un nombre suffisant d'échantillons du soluté de l'invention nécessaires jusqu'au prochain examen.

Au cours de cet essai, les examens cliniques ou fonctionnels ont laissé la place à l'interrogation du malade, qui devait répondre si le ronflement :
– avait disparu définitivement
– avait été supprimé avec récidive intermittente
– avait disparu pendant une période, puis était réapparu de façon plutôt régulière
– n'avait jamais disparu, mais était devenu moins important et moins régulier
– avait été amélioré, puis s'était ensuite péjoré.

Résultats :

Le nombre de dossiers exploitables après 3 mois de traitement a été faible au regard de la population totale de malades recrutés (80 malades). Ce résultat était attendu, du fait du nombre important de malades non revus. Les résultats ont pu être appréciés sur 48 malades parmi les 80 recrutés.
L'enquête ouverte, qui laissait l'initiative de l'appréciation au patient, a été préférée, au risque de diminuer le score final.
Cette approche a permis d'éviter des réponses trop enthousiastes chez certains malades.

Le tableau ci-dessous donne une indication des résultats obtenus après 3 mois de traitement avec le soluté nasal de l'invention.

| RESULTATS DE LA THERAPEUTIQUE | NOMBRE DE MALADES | % DE MALADES |
|---|---|---|
| Guérison | 9 | 19 |
| Amélioration | 31 | 64 |
| Echec | 8 | 17 |

La suppression totale du ronflement a été enregistrée chez 9 patients (19 %).
Parmi ces derniers, se trouvaient des ronfleurs occasionnels (6 patients) et des ronfleurs permanents (3

patients).

31 malades parmi les 48 ayant terminé l'essai, indiquaient une nette amélioration de leurs symptômes, soit par l'intermédiaire de leur partenaire de sommeil, soit parce que ce dernier le leur avait signalé après interrogation.

Dans cette population, se trouvent des personnes signalant, soit que l'intensité du bruit est moins gênante qu'auparavant (21 malades), soit que le bruit émis est différent ("bruit respiratoire" plutôt que "ronflement").

L'interrogation des malades n'a pas permis de déterminer si la seule tonalité du ronflement était modifiée, ou s'il s'agit de bruits d'origine différente (sybillances dans les cas de pneumopathies, par exemple).

Il est à remarquer que dans cette population de 31 malades améliorés par le traitement, 10 malades ont signalés avoir été guéris totalement pendant une période de temps, avant que ne reprenne le ronflement (33 % des malades).

8 patients ayant terminé l'essai ont enregistré un échec soit 17 % de la population ayant fait partie de l'échantillonnage sur lequel le produit a été évalué. Parfois l'amélioration a été temporaire, mais les partenaires des patients interrogés signalaient globalement une persistance du symptôme.

Dans 4 cas, il n'y a eu aucun changement du ronflement, parfois même une aggravation progressive du symptôme.

Dans tous les cas rapportés ci-dessus, le produit testé a toujours été bien toléré.

Il faut par ailleurs signaler, que certains malades ont rapporté des bénéfices thérapeutiques qui n'étaient pas prévus dans le cadre de l'objectif de cet essai clinique, comme :

– l'amélioration des relations conjugales ou familiales
– l'amélioration du sommeil (un grand nombre de malades indiquaient qu'ils "dormaient mieux", même quand ils nécessitaient une deuxième instillation au cours de la nuit).
– la disparition ou la réduction d'une somnolence au cours de la journée, qui est peut-être à rapprocher de la remarque précédente.
– l'amélioration de la respiration avant l'endormissement. Cette remarque n'est peut-être qu'une autre manière d'exprimer la diminution ou la disparition du ronflement.

Cet essai clinique mené sur 48 malades revus après 3 mois de traitement, a permis de déterminer l'efficacité du soluté nasal de l'invention dans le traitement symptomatique du ronflement.

83 % des malades (et/ou de leur partenaire conjugal) interrogés ont rapportés soit une guérison (19 %), soit une amélioration sensible de signes (64 %).

17 % de la population examinée n'a pas répondu au traitement. Cette inefficacité du traitement est vraisemblablement à rapprocher de ronchopathies plus graves, nécessitant éventuellement une intervention chirurgicale.

Dans tous les cas, la tolérance locale au traitement a été excellente.

## Revendications

1. Utilisation de polyanions sulfatés sous forme de polymères d'origine naturelle ou d'un de leurs sels sodiques pharmaceutiquement acceptables, pour la fabrication d'un soluté nasal destiné à la prévention ou au traitement du ronflement ou de ses manifestations sonores

2. Utilisation selon la revendication 1, caractérisé en ce que le polyanion sulfaté est un glycosaminoglycane sulfaté ou un sel sodique pharmaceutiquement acceptable.

3. Utilisation selon la revendication 2, caractérisé en ce que le glycosaminoglycane sulfaté est de type mucopolysaccharidique hydrosoluble.

4. Utilisation selon les revendications 1 et 3, caractérisé en ce que le soluté nasal comprend une solution aqueuse tamponnée, isotonique, contenant un conservateur compatible, de type mercuriel (nitrate ou borate phénylmercurique).

5. Composition adaptée pour une utilisation nasale sous forme de soluté caractérisée en ce que le principe actif est du type sulfate de chondroïtine sous forme de sel sodique.

6. Composition nasale selon la revendication 5, caractérisée en ce qu'elle renferme 0.1 % à 10 % de sel de sodique de sulfate de chondroïtine par rapport au poids total de la composition.

7. Utilisation ou composition selon l'une quelconque des revendications 1 à 6, caractérisée en ce que le soluté nasal contient en outre des constituants végétaux ou minéraux en concentration allopathique ou homéopathique.

8. Utilisation ou composition selon la revendication 7, caractérisée en ce que la composition contient des constituants homéopathiques tels que Belladonna, Bryonia, Sambuscus, Alumina et Plumbum, ou le mélange de ceux-ci, sous forme de dilutions hanemaniennes.

9. Utilisation thérapeutique selon les revendications 7 et 8, dans laquelle le polyanion sulfaté est le sulfate de chondroïtine sodique.

10. Composition selon l'une quelconque des revendications 5 ou 6, caractérisée en ce qu'elle contient en outre des constituants végétaux ou minéraux en concentration allopathique ou homéopathique, notamment Belladonna, Bryonia, Sambuscus, Alumina et Plumbum, ou le mélange de ceux-ci, sous forme de dilutions hanemaniennes.

**Patentansprüche**

1. Eine Verwendung von sulfatierten Polyanionen in Form von Polymeren natürlichen Ursprungs oder eines ihrer pharmazeutisch akzeptablen Natriumsalze für die Herstellung einer Nasenlösung, die zur Verhütung oder zur Behandlung des Schnarchens oder seiner geräuschvollen Äußerungen bestimmt ist.

2. Eine Verwendung gemäß Anspruch 1, dadurch gekennzeichnet, daß es sich bei dem sulfatierten Anion um ein sulfatiertes Glykosaminoglykan oder um ein pharmazeutisch akzeptables Natriumsalz handelt.

3. Eine Verwendung gemäß Anspruch 2, dadurch gekennzeichnet, daß es sich bei dem sulfatierten Glykosaminoglykan um ein wasserlösliches Mucopolysaccharid handelt.

4. Eine Verwendung gemäß den Ansprüchen 1 und 3, dadurch gekennzeichnet, daß die Nasenlösung eine isotonische wäßrige Pufferlösung mit einem verträglichen Konservierungsmittel auf der Grundlage von Quecksilber (Phenylquecksilbernitrat oder -borat) enthält.

5. Eine für Anwendungen auf die Nase geeignete Mischung in Form einer Lösung, gekennzeichnet dadurch, daß der Wirkstoff von der Art des Chondroitinsulfats in Form eines Natriumsalzes ist.

6. Eine Mischung für die Nase gemäß Anspruch 5, gekennzeichnet dadurch, daß sie 0,1 % bis 10 % Chondroitinsulfatnatriumsalz im Vergleich zu dem Gesamtgewicht der Mischung enthält.

7. Eine Verwendung oder eine Mischung gemäß einem beliebigen der Ansprüche 1 bis 6, gekennzeichnet dadurch, daß die Nasenlösung außerdem pflanzliche oder mineralische Bestandteile in einer allopathischen oder homöopathischen Konzentration enthält.

8. Eine Verwendung oder eine Mischung gemäß Anspruch 7, dadurch gekennzeichnet, daß die Mischung homöopathische Bestandteile wie zum Beispiel Belladonna, Bryonia, Sambuscus, Alumina und Plumbum oder deren Gemisch in Form von Hahnemann schen Verdünnungen enthält.

9. Eine therapeutische Verwendung gemäß den Ansprüchen 7 und 8, bei dem es sich bei dem sulfatierten Polyanion um Natriumchondroitinsulfat handelt.

10. Eine Mischung gemäß einem beliebigen der Ansprüche 5 oder 6, gekennzeichnet dadurch, daß sie außerdem pflanzliche oder mineralische Bestandteile in einer allopathischen oder homöopathischen Konzentration enthält, und zwar insbesondere Belladonna, Bryonia, Sambuscus, Alumina und Plumbum oder deren Gemisch in Form von Hahnemann schen Verdünnungen.

## Claims

1. Use of sulphated polyanions in the form of polymers of natural origin, or one of their sodium salt pharmaceutically speaking acceptable, in order to manufacture a nasal solution aimed at the prevention or treatment of snoring or its noisy outward symptoms.

2. Use according to claim n° 1, characterized in the fact that the sulphated polyanion is a sulphated glycosaminoglycane or a sodium salt pharmaceutically speaking acceptable.

3. Use according to claim 2, characterized by the fact that the sulphated glycosaminoglycane is of the water soluble mucopolysaccharide type.

4. Use according to claims 1 to 3, characterized by the fact that the nasal solution is a buffered aqueous solution with isotonic properties, containing a compatible preservative of a mercury type (phenylmercury nitrate or borate).

5. Composition adapted for a nasal use in the form of a solution, characterized in that the principle active agent is chondroitin sulphate in the form of sodium salt.

6. Nasal composition according to claim 5, characterized by the fact that it contains 0.1 to 10 % of sodic chondroitin sulphate in relation to the total weight of the nasal solution.

7. Use or composition according to any one of the claims from 1 to 6, characterized by the fact that it contains allopathic or homeopathic plant or mineral constituents combined with sodic chondroitin sulphate.

8. Use or composition according to claim 7, characterized by the fact that the composition contains homeopathic constituents such as Belladonna, Bryonia, Sambucus, Aluminia and Plumbum, or a mixture of the latter, in the form of Hahnemanian (homeopathic) dilutions.

9. Therapeutical composition according to claims 7 and 8, in which the sulphated polyanion is the sodic chondroitin sulphate.

10. Composition according to any one of the claims 5 or 6, characterized by the fact that it contains, besides, allopathic or homeopathic plant or mineral constituents, such as Belladonna, Bryonia, Sambucus, Aluminia and Plumbum, or a mixture of the latter, in the form of Hahnemanian (homeopathic) dilutions.